(19) Europäisches Patentamt
European Patent Office
Office européen des brevets

(11) Veröffentlichungsnummer: **0 000 174**
**B1**

(12) # EUROPÄISCHE PATENTSCHRIFT

(45) Veröffentlichungstag der Patentschrift: **30.12.81**

(21) Anmeldenummer: **78100206.8**

(22) Anmeldetag: **21.06.78**

(51) Int. Cl.³: **C 07 C 103/82**, C 07 D 207/04, C 07 D 213/40, C 07 D 239/90, C 07 D 401/06, C 07 D 403/06, A 61 K 31/505, A 61 K 31/40, A 61 K 31/44, A 61 K 31/165

(54) **Aminobenzoesäurederivate, Verfahren zu ihrer Herstellung und Arzneimittel enthaltend solche Aminobenzoesäurederivate.**

(30) Priorität: **04.07.77 DE 2730174**

(43) Veröffentlichungstag der Anmeldung: **10.01.79 Patentblatt 79/1**

(45) Bekanntmachung des Hinweises auf die Patenterteilung: **30.12.81 Patentblatt 81/52**

(84) Benannte Vertragsstaaten: **BE CH DE FR GB LU NL SE**

(56) Entgegenhaltungen:
**DE-A-1 595 915**
**DE-A-1 957 319**
**DE-A-2 612 321**
**DE-A-2 623 228**
**US-A-3 192 214**

(73) Patentinhaber: **Ludwig Merckle, K.G. chem. pharm. Fabrik, Dr.-Georg-Spohn-Strasse 7, D-7902 Blaubeuren (DE)**

(72) Erfinder: **Metz, Gunter, Dr., Auf dem Rucken 29, D-7902 Blaubeuren (DE)**
Erfinder: **Specker, Manfred, Dr., Weilerhalde 32, D-7902 Blaubeuren (DE)**

(74) Vertreter: **Lederer, Franz, Dr. et al, Patentanwälte Lederer, Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 000 174 B1

ACTORUM AG.

# Aminobenzoesäurederivate, Verfahren zu ihrer Herstellung und Arzneimittel enthaltend solche Aminobenzoesäurederivate

Gegenstand der Erfindung sind neue Aminobenzoesäurederivate der allgemeinen Formel

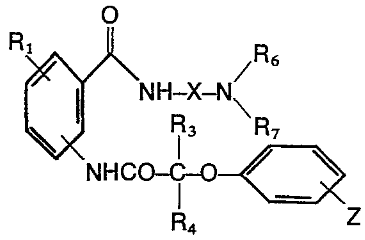

II

oder

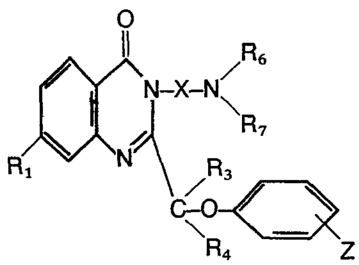

III

worin bedeuten

$R_1$ Wasserstoff, Chlor, Hydroxy, Acetoxy oder $C_1$–$C_3$-Alkoxy;

$R_3$ Wasserstoff oder Methyl,

$R_4$ Wasserstoff, $C_1$–$C_3$-Alkyl oder mit Z substituiertes Phenoxy, mit der Massgabe, dass in Formel III mindestens einer der Reste $R_3$ und $R_4$ ein Wasserstoffatom darstellt,

Z Halogen oder Trifluormethyl,

X $C_1$–$C_3$-Alkylen,

$R_6$ und $R_7$ Äthyl oder zusammen mit dem Stickstoffatom eine

N-Pyrrolidinyl ( 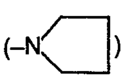 ),

N-Piperidinyl ( 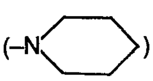 ),

N-Äthyl-2-pyrrolidinyl ( 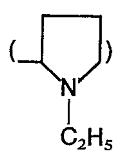 ) oder

3-Pyridinyl ( 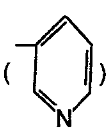 ) -Gruppe

sowie deren mit gegebenenfalls Phenyl-substituierten $C_1$–$C_4$-Alkyl- oder $C_1$–$C_4$-Alkenyl-Gruppen enthaltenden Quaternisierungsmitteln oder pharmazeutisch verträglichen Säuren erhaltenen Derivate.

Als Halogenatome im Rest Z kommen Fluor, Chlor, Brom und Jodatome, vorzugsweise Chlor- und Fluoratome in Frage. Die p- und o-Stellung, insbesondere die p-Stellung, wird bevorzugt. Die Trifluormethylgruppe steht bevorzugt in m-Stellung.

Beispiele für geeignete Quaternisierungsreste sind Halogenalkylreste wie der Methojodid-, Äthojodidrest, der Butylbromid- und der Phenylpropenylbromidrest.

Die erfindungsgemässen Aminobenzoesäurederivate können dadurch hergestellt werden, dass man eine Carbonsäure der allgemeinen Formel

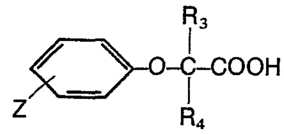

IV

worin Z, $R_3$ und $R_4$ die oben angegebenen Bedeutungen besitzen oder ein reaktionsfähiges Derivat dieser Carbonsäure mit einer Aminobenzoesäure der allgemeinen Formel

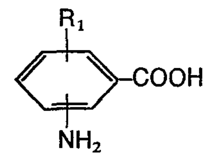

V

in der $R_1$ die oben angegebene Bedeutung besitzt oder deren reaktionsfähigem Derivat zur Reaktion bringt und die erhaltene Aminobenzoesäure der allgemeinen Formel

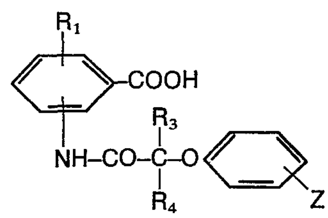

VI

oder deren reaktionsfähiges Derivat mit einem Diamin der allgemeinen Formel

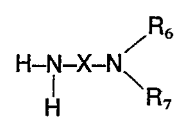

VII

worin X, $R_6$ und $R_7$ die oben angegebene Bedeutung haben, umsetzt und sodann gegebenenfalls quaternisiert.

Ein weiteres Verfahren besteht darin, dass die Aminobenzoesäure der allgemeinen Formel (V) oder deren reaktionsfähiges Derivat, zweckmässig unter Einführung eines Formyl- oder Acetylrestes als Schutzgruppe für den $NH_2$-Rest, zunächst mit dem Diamin der allgemeinen Formel (VII) umgesetzt wird und nach Entfernen der Schutzgruppe mit der Carbonsäure der allgemeinen Formel (IV) oder deren reaktionsfähigem Derivat zur Reaktion gebracht wird.

Sofern in der allgemeinen Formel (II) die Amidgruppe in o-Stellung steht, wird das Verfahren zweckmässig derart abgeändert, dass Isatosäureanhydrid als reaktionsfähiges Derivat der Aminobenzoesäure der allgemeinen Formel (V), gegebenenfalls substituiert mit $R_1$, mit dem Diamin der allgemeinen Formel (VII) in einem geeigneten Lösungsmittel umgesetzt wird und das hierbei erhaltene Aminobenzoesäurederivat der allgemeinen Formel

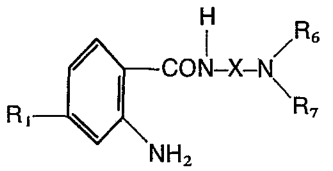

(VIII)

mit der Carbonsäure der allgemeinen Formel (IV) oder deren reaktionsfähigem Derivat zur Reaktion gebracht wird.

Geeignete Säurederivate sind z.B. Säurechloride, Säureanhydride, Ester sowie die durch Umsetzung der Carboxylgruppe mit Halogenameisensäureestern zugänglichen Alkylkohlensäureester resp. Alkylkohlensäureanhydride.

Die direkte Umsetzung der Carbonsäure (IV) mit der Aminobenzoesäure (V) oder die direkte Umsetzung der Aminobenzoesäure (VI) mit dem Diamin (VII) erfolgt bevorzugt in aromatischen Kohlenwasserstoffen oder Halogenkohlenwasserstoffen unter Erwärmung auf Rückflusstemperatur bei gleichzeitigem Einsatz von wasserabspaltenden Komponenten, wie z.B. Phosphoroxychlorid, Phosphortrichlorid, Phosphorpentachlorid, Dicyclohexylcarbodiimid.

Die Umsetzung der Säurederivate mit dem Diamin (VII) erfolgt bevorzugt in Halogenkohlenwasserstoffen oder Äthern bei Raumtemperatur oder unter Erwärmen bis auf Siedetemperatur.

Bei der Umsetzung der Carbonsäure mit einem Chlorameisensäureester wird zweckmässig das gebildete Alkylkohlensäureanhydridderivat nicht isoliert, sondern in einem Einstufenverfahren direkt mit dem Diamin (VII) zur Reaktion gebracht.

Die erhaltenen basischen Aminobenzoesäurederivate der allgemeinen Formel (II) oder (III) können durch Umsetzung mit pharmazeutisch gebräuchlichen Säuren, wie z.B. Halogenwasserstoffsäuren, Citronensäure, Fumarsäure, Salizylsäure, Nicotinsäure sowie mit den Säuren der allgemeinen Formel (IV) und (V) in die entsprechenden Salze überführt werden oder durch Umsetzung mit Halogenalkanen quaternisiert werden.

Die cyclisierten Aminobenzoesäurederivate der allgemeinen Formel (III) sind darstellbar durch Erhitzen der entsprechenden o-substituierten Aminobenzoesäuren (II) in geeigneten Lösungsmitteln oder lösungsfrei, oder direkt erhältlich bei der Umsetzung der Aminobenzoesäure der allgemeinen Formel (VI) oder deren reaktionsfähigem Derivat mit dem Diamin der allgemeinen Formel (VII). Bei beiden Herstellungsverfahren ist einschränkend anzuführen, dass infolge sterischer Hinderung bei den cyclisierten Verbindungen der allgemeinen Formel (III) wenigstens einer der beiden Reste $R_3$ und $R_4$ ein Wasserstoffatom darstellen muss.

Die erfindungsgemässen Verbindungen besitzen insbesondere eine ausgezeichnete antilipämische Aktivität bei guter Verträglichkeit.

Die therapeutische Wirksamkeit der erfindungsgemässen Verbindungen nach Beispielen 2, 39, 69 und 75 wurde im Tierversuch an weiblichen Ratten gegen die bekannten Antilipaemica 2-(p-Chlorphenoxy)-2-methyl-propionsäure (Clofibrinsäure), deren Äthylester (Clofibrat) sowie gegen eine der Ausgangsverbindungen der allgemeinen Formel (VI), nämlich 2-[2-(p-Chlorphenoxy)-2-methylpropionamido]-benzoesäure (Ref. 1) getestet. Hierbei wurden die Verbindungen bzw. das Vehikel (1%ige Tragantlösung) über einen Versuchszeitraum von 14 Tagen mittels Schlundsonde verabfolgt, 24 Stunden nach der letzten Gabe wurden die Blutproben zur Bestimmung der Serumcholesterin- und Serumtriglyzeridspiegel entnommen.

In einer ersten Serie wurde die Wirksamkeit an normolipaemischen Ratten (10 Tiere/Gruppe) mit einem Körpergewicht von 50 bis 60 g untersucht, die über den gesamten Versuchszeitraum eine standardisierte, pelletierte Labordiät (Normaldiät) erhielten. Die Diät bestand im wesentlichen aus Rohproteinen und Kohlehydraten bei einem Rohfettgehalt von max. 3,9%, angereichert mit Vitaminen, Mineralstoffen und Aminosäuren.

Die Ergebnisse dieser Serie sind in der Tabelle 1 zusammengefasst. Die angegebenen Werte der prozentualen Veränderung (±%) beziehen sich auf den jeweiligen Gruppenmittelwert (mg%) der Kontrollgruppe, der an 20 Tieren bestimmt wurde. Die Signifikanz p ist ebenfalls auf die Kontrollwerte bezogen.

Tabelle 1
Antilipämische Aktivität unter Normaldiät

| Beispiel | mg/kg | Cholesterin ± % | Triglyzeride ± % |
|---|---|---|---|
| 39 | 250 | −0,33 | −46,0*** |
| Clofibrinsäure | 100 | +1,1 | −20,8 |
| | 250 | −6,6 | −26,5* |
| Ref. 1 | 100 | +2,1 | + 0,8 |
| | 300 | +0,9 | + 2,9 |

* $p < 0,05$
*** $p < 0,001$

In einer zweiten Studie wurde die Wirksamkeit an hypercholesterinämischen Ratten gemäss den Angaben von Berger et al., Proc. Soc. Exp. Biol. 132, 253 (1969) untersucht. Die anfangs normolipämischen Tiere erhielten zur Erzielung einer artefiziellen Hyperlipidaemie die oben beschriebene Normaldiät in pulverisierter Form, die mit 2% Cholesterin sowie 1% Cholsäure versetzt wurde. Die Ergebnisse dieser Studie sind in Tabelle 2 zusammengefasst, wobei sich die angegebenen Veränderungen ebenfalls auf den Gruppenmittelwert der Kontrollgruppe beziehen.

Tabelle 2
Antilipämische Aktivität unter Hypercholesterindiät

| Beispiel | mg/kg | Cholesterin ± % | Triglyzeride ± % |
|---|---|---|---|
| 39 | 250 | −63,6*** | − 6,7 |
| 69 | 131 | −35,2* | + 1,5 |
| 2 (HCl) | 279 | −12,3 | − 6,4 |
| 75 (HCl) | 275 | − 3,5 | −29,4 |
| Clofibrinsäure | 100 | + 6,7 | − 8,2 |
| | 250 | + 9,5 | −17,4 |
| Clofibrat | 250 | − 3,0 | −12,5 |
| Ref. 1 | 100 | + 3,2 | −25,7 |
| | 300 | + 5,0 | +25,5 |

* $p < 0,05$
** $p < 0,01$
*** $p < 0,001$

Berücksichtigt man, dass in den getesteten erfindungsgemässen Verbindungen bezüglich der Lipidsenkung der Clofibrinsäurerest das aktive Prinzip darstellt und dass unter den besonders erschwerten Bedingungen der Hypercholesterintests die dosierte Menge der Verbindungen nach Beispiel 2, 39 und 75 einer Verabreichung von 128 mg/kg Clofibrinsäure, die der Verbindung nach Beispiel 69 sogar nur 64 mg/kg Clofibrinsäure entspricht, so ergibt sich hieraus im Vergleich zu Clofibrinsäure und Clofibrat ein wesentlich höherer therapeutischer Index. Überraschend hingegen ist völlige Inaktivität der getesteten Referenzverbindung der allgemeinen Formel (VI) (Ref. 1). Übereinstimmend mit den Ergebnissen der Tabelle 2 dürfte als gesichert geltend, dass die graduelle Aktivität der Verbindungen hauptsächlich vom basischen Rest gemäss allgemeiner Formel (VII) bestimmt wird.

Wie ein pharmakologisches Screening an ausgewählten Verbindungen der allgemeinen Formel (II) und (III) zeigte, besitzen diese neben antilipämischer Aktivität noch wertvolle therapeutische Eigenschaften. So hemmen die Verbindungen nach Beispiel 12 und 51 die Blutblättchenaggregation mit einer Aktivität, die der von Adenosin und Acetylsalicylsäure überlegen ist. Die Verbindungen nach Beispiel 79 und 80 zeigen insbesondere antiarrhytmische und cardiotrope Eigenschaften sowie eine ausgeprägte $\beta$-adrenergische Hemmung, die den bekannten Referenzverbindungen, wie Practalol oder Prinodolol, wirkungsgleich oder überlegen ist.

Die erfindungsgemässen Arzneimittel enthalten eine oder mehrere Aminobenzoesäurederivate der allgemeinen Formel (II) oder (III) als Wirkstoff. Die Anwendung erfolgt vorzugsweise oral, z.B. in Form von Tabletten oder Kapseln, die gegebenenfalls übliche pharmazeutische Trägerstoffe und Hilfsmittel, z.B. Laktose, Stärke, Talk und Magnesiumstearat, enthalten. Zur Anwendung in Injektionslösungen sind insbesondere deren pharmazeutische verträglichen Salze geeignet.

Die Verabreichung der erfindungsgemässen Verbindungen erfolgt, je nach dem Fall, in oralen oder rektalen Tagesdosen von 250 bis 1500 mg, vorzugsweise 500 bis 750 mg, in den üblichen pharmazeutischen Formen oder als Injektionslösung in Tagesdosen von 50 bis 250 mg, vorzugsweise 100 bis 200 mg.

Die Herstellung der erfindungsgemässen Verbindungen wird anhand folgender Beispiele beschrieben:

Beispiel 1

13,7 g (0,1 Mol) 3-Aminobenzoesäure werden unter Zusatz von 10 g (0,1 Mol) Triäthylamin in 150 ml Chloroform suspendiert, mit 20,5 g (0,1 Mol) p-Chlorphenoxyacetylchlorid versetzt und 5 Stunden unter Rückfluss erwärmt. Nach dem Erkalten wird das gebildete Kristallisat abgesaugt. Es werden 23,3 g (76,5%) 3-(p-Chlorphenoxyacetamido)-benzoesäure vom Fp 208 °C erhalten.

15,24 g (0,05 Mol) dieser Amidobenzoesäure werden in 100 ml Toluol unter Zusatz von 6,4 g (0,55 Mol) Diäthylaminoäthylamin suspendiert und mit 7,7 g (0,05 Mol) Phosphoroxychlorid versetzt. Der Ansatz wird anschliessend 6 Stunden unter Rückfluss erwärmt. Nach dem Erkalten wird die Lösung mit Wasser geschüttelt und mit verdünnter Natronlauge alkalisiert. Nach dem Abtrennen der alkalischen Phase wird die Lösung nochmals mit Wasser gewaschen. Der nach dem Abdestillieren des Lösungsmittels verbleibende

Rückstand wird aus wenig Diisopropyläther kristallisiert, wobei 16,8 g (83,2%) 3-(p-Chlorphenoxyacetamido)-N-(2-diäthylaminoäthyl)-benzamid vom Fp 100 °C erhalten werden.
Elementaranalyse:
$C_{21}H_{26}ClN_3O_3$ (403,9)

| | | | |
|---|---|---|---|
| Ber.: | C 62,43 | H 6,49 | N 10,40 |
| Gef.: | C 62,33 | H 6,56 | N 10,03 |

Hydrochlorid Fp 92 °C.

Beispiel 2

64 g (0,2 Mol) 4-[2-(p-Chlorphenoxy)-2-methyl-propionamido]-benzoesäure vom Fp 191 °C werden mit 24 g Thionylchlorid in 200 ml Toluol umgesetzt und 6 Stunden auf 100 °C erwärmt. Das nach dem Erkalten gebildete Kristallisat wird abgesaugt, wobei 45 g (66,3%) des entsprechenden Säurechlorids vom Fp 148° erhalten werden. 17,8 g (0,052 Mol) dieses Säurechlorids werden in 150 ml Chloroform gelöst und mit 6 g 1-(2-Aminoäthyl)-pyrrolidin unter Erwärmen umgesetzt. Die mit verdünnter Natronlauge und Wasser gewaschene Chloroformlösung wird eingedampft und der Rückstand aus Aceton kristallisiert. Es werden 14,5 g (64,3%) 4-[2-(p-Chlorphenoxy)-2-methyl-propionamido]-N-(1-äthylpyrrolidinyl-2-methyl)-benzamid vom Fp 180° erhalten.
Elementaranalyse:
$C_{23}H_{28}ClN_3O_3$(429,9)

| | | | |
|---|---|---|---|
| Ber.: | C 64,25 | H 6,56 | N 9,77 |
| Gef.: | C 64,36 | H 6,62 | N 9,91 |

Hydrochlorid Fp 191 °C.

Beispiel 3

16,7 g (0,05 Mol) 2-[2-(p-Chlorphenoxy)-2-methyl-propionamido]-benzoesäure vom Fp 199 °C werden in 60 ml Toluol suspendiert und nacheinander mit 2,05 g Phosphortrichlorid und 5,9 g 2-Diäthylaminoäthylamin versetzt. Der Ansatz wird noch 5 Stunden unter Rückfluss erwärmt und nach dem Erkalten mit verdünnter Natronlauge und Wasser gewaschen.

Nach dem Abdestillieren des Lösungsmittels werden 10 g (42%) 2-[2-(p-Chlorphenoxy)-2-methyl-propionamido)-N-(2-diäthylaminoäthyl)-benzamid als Öl erhalten.
Der ölige Rückstand wird in Diisopropyläther gelöst und mit isopropanolischer Salzsäure bis pH 3 versetzt. Das hierbei gebildete Hydrochlorid wird abgesaugt und zeigt nach dem Trocknen einen Fp von 149 °C. Die Titration mit 0,1N $HClO_4$ in Eisessig unter Zusatz von Quecksilberacetet ergibt einen Gehalt von 102,8%.

Beispiel 4

16,3 g (0,1 Mol) Isatosäureanhydrid werden in 100 ml Toluol suspendiert und mit 11,6 g (0,1 Mol) 2-Diäthylaminoäthylamin versetzt. Die erhaltene Suspension wird 4 Stunden gerührt, wobei unter milder exothermer Reaktion und Abspaltung von $CO_2$ allmählich Lösung eintritt. Nach dem Stehen über Nacht wird die filtrierte Lösung mit verdünnter Salzsäure ausgeschüttelt. Die salzsaure wässrige Phase wird mit NaOH alkalisiert und mit Chloroform extrahiert. Nach dem Abdampfen des Lösungsmittels werden 20,8 g (88,4%) N-(2-Diäthylaminoäthyl)-2-amino-benzamid als Öl erhalten.

10 g (0,042 Mol) dieses Amids werden in Chloroform mit 8,6 g (0,042 Mol) p-Chlorphenoxyessigsäurechlorid umgesetzt. Die mit verdünnter Natronlauge sowie Wasser gewaschene Chloroformlösung wird eingedampft, wobei 12,8 g (75,7%) 2-(p-Chlorphenoxyacetamido)-N-(2-diäthylaminoäthyl)-benzamid vom Fp 109 °C erhalten werden.
Citrat Fp 111 °C/Maleinat Fp 134 °C.

Beispiel 5

30,6 g (0,2 Mol) 5-Amino-2-hydroxy-benzoesäure werden gemäss Verfahren nach Beispiel 1 mit 41 g (0,2 Mol) p-Chlorphenoxyacetylchlorid umgesetzt. Es werden 62,7 g (97,5%) 5-(p-Chlorphenoxyacetamido)-2-hydroxybenzoesäure vom Fp 227 °C erhalten.

Die Umsetzung mit Acetanhydrid ergibt 5-(p-Chlorphenoxyacetamido)-2-acetylbenzoesäure vom Fp 198 °C in einer Ausbeute von 58,5%.

36,4 g (0,1 Mol) dieser Acetylbenzoesäure werden in 200 ml Tetrahydrofuran unter Zusatz von 12,0 g (0,12 Mol) Triäthylamin suspendiert. Bei einer Temperatur von 5 bis 10 °C werden 13,0 g (0,12 Mol) Chlorameisensäureäthylester zugesetzt und der Ansatz noch 3 Stunden gerührt, wobei nach 2 Stunden die Kühlung entfernt wird. 12,8 g (0,1 Mol) 1-Äthyl-2-(aminomethyl)-pyrrolidin werden unter Rühren zugesetzt. Nach dem Stehen über Nacht wird das Lösungsmittel weitgehend im Vakuum eingedampft und der Rückstand mit Wasser zerlegt.

Das hierbei abgeschiedene zähe Öl wird mit Chloroform aufgenommen und mit verdünnter Natronlauge und Wasser gewaschen. Der nach dem Eindampfen verbleibende ölige Rückstand kristallisiert aus wenig Diisopropyläther, wobei 24,7 g (52,2%) 5-(p-Chlorphenoxyacetamido)-2-acetyl-N-(1-äthylpyrrolidinyl-2-methyl)-benzamid vom Fp 139 °C erhalten werden. Die Gehaltsbestimmung durch Titration mit 0,1N $HClO_4$ in Eisessig zeigt einen Gehalt von 103,4%.

Beispiel 6

1 g (21 mMol) des nach Beispiel 5 hergestellten Benzamids wird in wässriger Natronlauge unter Zusatz von wenig Äthanol 15 Minuten auf etwa 80 °C erwärmt und anschliessend mit verdünnter Salzsäure angesäuert. Nach Extraktion mit Chloroform werden nach dem Abdampfen der Lösungsmittel 0,8 g (88,2%) 5-(p-Chlorphenoxyacetamido)-2-hydroxy-N-(1-äthylpyrrolidinyl-2-methyl)-benzamid Hydrochlorid vom Fp 112 °C erhalten.

Das durch Behandeln mit verdünnter Natronlauge aus Hydrochlorid zugängliche Benzamid zeigt einen Fp von 87 °C.

Entsprechend den Verfahren nach Beispiel 1 bis 6 wurden weitere Verbindungen hergestellt, die zusammen mit den Verbindungen der vorgehenden Beispiele in den nachstehenden Tabellen tabellarisch erfasst sind.

| Bei-spiel | allge-meine Formel | $R_1$ | Phenoxy-amid-Gruppe in Stellung | $R_3$ | $R_4$ | Z | 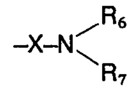 | Quaternisierung bzw. Salzbildung | | Fp °C |
|---|---|---|---|---|---|---|---|---|---|---|
| 1 | II | H | 3 | H | H | p-Cl | 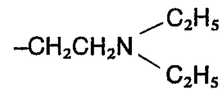 | –<br>H | –<br>Cl | 100<br>92 |
| 2 | II | H | 4 | $CH_3$ | $CH_3$ | p-Cl | 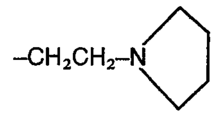 | –<br>H | –<br>Cl | 180<br>191 |
| 3 | II | H | 2 | $CH_3$ | $CH_3$ | p-Cl | 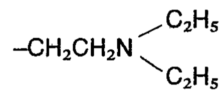 | –<br>H | –<br>Cl | ölig<br>149 |
| 4 | II | H | 2 | H | H | p-Cl | 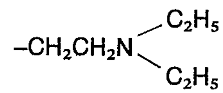 | –<br>H<br>H | –<br>Citrat<br>Maleinat | 109<br>111<br>134 |
| 5 | II | 2-$OCOCH_3$ | 5 | H | H | p-Cl | 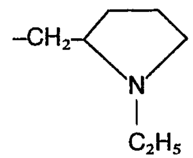 | – | – | 139 |
| 6 | II | 2-OH | 5 | H | H | p-Cl | ,, | –<br>H | –<br>Cl | 87<br>112 |
| 7 | II | 2-Cl | 4 | H | H | p-Cl | 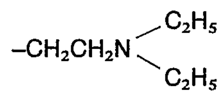 | –<br>H | –<br>Citrat | 133<br>124 |
| 8 | II | 2-Cl | 4 | H | H | p-Cl | 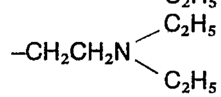 | $CH_3$ | J | 229 |
| 9 | II | 2-Cl | 4 | H | H | p-Cl | 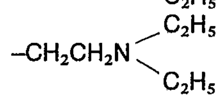 | $CH_3$ | Cl | 117 |
| 10 | II | H | 2 | H | H | p-Cl | 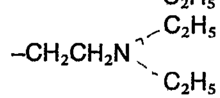 | $C_6H_5CH{=}CH–CH_2$ | Br | 182 |
| 11 | II | H | 4 | H | H | p-Cl | 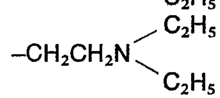 | –<br>H | –<br>Citrat | 168<br>127 |
| 12 | II | 4-Cl | 3 | H | H | p-Cl | 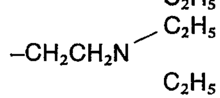 | –<br>H | –<br>Citrat | 170<br>148 |

| | | | | | | | | | | |
|---|---|---|---|---|---|---|---|---|---|---|
| 13 | II | 2-OH | 5 | H | H | p-Cl | $-CH_2CH_2N(C_2H_5)_2$ | – | – | 120 |
| 14 | II | $2\text{-}OCOCH_3$ | 5 | H | H | p-Cl | $-CH_2CH_2N(C_2H_5)_2$ | – | – | 127 |
| 15 | II | $2\text{-}OCH_3$ | 5 | H | H | p-Cl | $-CH_2CH_2N(C_2H_5)_2$ | – | – | 148 |
| 16 | II | H | 4 | H | H | p-J | $-CH_2CH_2N(C_2H_5)_2$ | –<br>H | –<br>Cl | 166<br>136 |
| 17 | II | H | 4 | H | H | p-Br | $-CH_2CH_2N(C_2H_5)_2$ | –<br>H | –<br>Citrat | 166<br>124 |
| 18 | II | H | 4 | H | $OC_6H_4\text{-}p\text{-}Cl$ | p-Cl | $-CH_2CH_2N(C_2H_5)_2$ | –<br>H | –<br>Citrat | 182<br>155 |
| 19 | II | H | 4 | H | H | m-Cl | $-CH_2CH_2N(C_2H_5)_2$ | – | – | 100 |
| 20 | II | H | 4 | H | H | p-F | $-CH_2CH_2N(C_2H_5)_2$ | –<br>H | –<br>Cl | 150<br>131 |
| 21 | II | H | 4 | H | H | p-F | $-CH_2CH_2N(C_2H_5)_2$ | $CH_2CH_2CH_2CH_3$ | Br | 143 |
| 22 | II | H | 4 | H | H | $m\text{-}CF_3$ | $-CH_2CH_2N(C_2H_5)_2$ | –<br>H | –<br>Cl | 130<br>195 |
| 23 | II | H | 4 | H | H | o-Cl | $-CH_2CH_2N(C_2H_5)_2$ | –<br>H | –<br>Citrat | 174<br>124 |
| 24 | II | 5-Cl | 2 | H | H | p-Cl | $-CH_2CH_2N(C_2H_5)_2$ | –<br>H<br>H | –<br>Citrat<br>Fumarat | 140<br>134<br>157 |
| 25 | II | 5-Cl | 2 | H | H | p-Cl | $-CH_2CH_2N(C_2H_5)_2$ | $CH_3$ | J | 216 |
| 26 | II | $2\text{-}OCOCH_3$ | 4 | H | H | p-Cl | $-CH_2CH_2N(C_2H_5)_2$ | – | – | 234 |
| 27 | II | 2-OH | 4 | H | H | p-Cl | $-CH_2CH_2N(C_2H_5)_2$ | – | – | 164 |

| Beispiel | allgemeine Formel | $R_1$ | Phenoxy-amid-Gruppe in Stellung | $R_3$ | $R_4$ | Z | $-X-N{<}{R_6 \atop R_7}$ | Quaternisierung bzw. Salzbildung | | Fp °C |
|---|---|---|---|---|---|---|---|---|---|---|
| 28 | II | 2-OH | 4 | H | H | p-Cl | $-CH_2CH_2N{<}{C_2H_5 \atop C_2H_5}$ | $CH_3$ | J | 158 |
| 29 | II | 2-$OCH_3$ | 4 | H | H | p-Cl | $-CH_2CH_2N{<}{C_2H_5 \atop C_2H_5}$ | – | – | 192 |
| 30 | II | 4-$OCH_3$ | 3 | H | H | p-Cl | $-CH_2CH_2N{<}{C_2H_5 \atop C_2H_5}$ | – | – | 184 |
| | | | | | | | | H | Cl | 184 |
| 31 | II | 4-$OCH_3$ | 3 | H | H | p-Cl | $-CH_2CH_2N{<}{C_2H_5 \atop C_2H_5}$ | $CH_3$ | J | 211 |
| 32 | II | H | 4 | $CH_3$ | H | p-Cl | $-CH_2CH_2N{<}{C_2H_5 \atop C_2H_5}$ | – | – | 135 |
| | | | | | | | | H | Citrat | 151 |
| 33 | II | H | 4 | H | $CH_3$ | p-Cl | $-CH_2CH_2N{<}{C_2H_5 \atop C_2H_5}$ | $C_6H_5CH=CHCH_2$ | Br | 117 |
| 34 | II | H | 3 | $CH_3$ | H | p-Cl | $-CH_2CH_2N{<}{C_2H_5 \atop C_2H_5}$ | – | – | 141 |
| | | | | | | | | H | Cl | 155 |
| 35 | II | H | 3 | $CH_3$ | H | p-Cl | $-CH_2CH_2N{<}{C_2H_5 \atop C_2H_5}$ | $CH_3$ | J | 139 |
| 36 | II | H | 2 | $CH_3$ | H | p-Cl | $-CH_2CH_2N{<}{C_2H_5 \atop C_2H_5}$ | – | – | ölig |
| | | | | | | | | H | Cl | 171 |
| 37 | II | 5-Cl | 2 | $CH_3$ | H | p-Cl | $-CH_2CH_2N{<}{C_2H_5 \atop C_2H_5}$ | – | – | ölig |
| | | | | | | | | H | Cl | 146 |
| 38 | II | 2-Cl | 4 | $CH_3$ | H | p-Cl | $-CH_2CH_2N{<}{C_2H_5 \atop C_2H_5}$ | $CH_3$ | J | 55 (sint.) |
| 39 | II | H | 4 | $CH_3$ | $CH_3$ | p-Cl | $-CH_2CH_2N{<}{C_2H_5 \atop C_2H_5}$ | – | – | 162 |
| | | | | | | | | H | $A^1$ | 128 |
| | | | | | | | | H | $A^2$ | 52 |
| | | | | | | | | H | Nicotinat | 291 |

| | | | | | | | | | | |
|---|---|---|---|---|---|---|---|---|---|---|
| 40 | II | H | 3 | $CH_3$ | $CH_3$ | p-Cl | 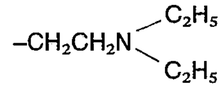 | –<br>H | –<br>Cl | ölig<br>146 |
| 41 | II | 5-Cl | 2 | $CH_3$ | $CH_3$ | p-Cl | 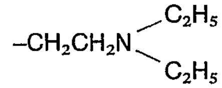 | –<br>H | –<br>Cl | ölig<br>167 |
| 42 | II | 2-Cl | 4 | $CH_3$ | $CH_3$ | p-Cl | 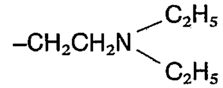 | –<br>H<br>H | –<br>Cl<br>Citrat | 116<br>160<br>122 |
| 43 | II | 4-$OCH_3$ | 3 | $CH_3$ | $CH_3$ | p-Cl | 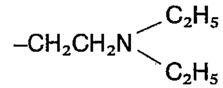 | –<br>H | –<br>Cl | 79<br>160 |
| 44 | II | 2-Cl | 5 | $CH_3$ | $CH_3$ | p-Cl | 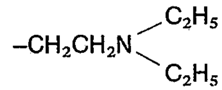 | –<br>H | –<br>Cl | 103<br>175 |
| 45 | II | 2-$OCOCH_3$ | 4 | $CH_3$ | $CH_3$ | p-Cl | 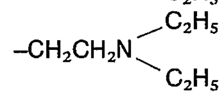 | – | – | ölig |
| 46 | II | 2-OH | 4 | $CH_3$ | $CH_3$ | p-Cl | 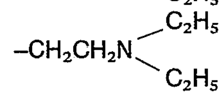 | – | – | ölig |
| 47 | II | 2-OH | 4 | $CH_3$ | $CH_3$ | p-Cl | 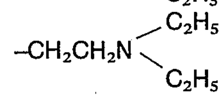 | $CH_3$ | J | 124 |
| 48 | II | 2-Cl | 5 | H | H | p-Cl | 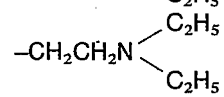 | –<br>H | –<br>Cl | 131<br>228 |
| 49 | II | 2-Cl | 5 | H | H | p-Cl | 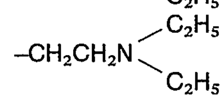 | $C_2H_5$ | J | 110 |
| 50 | II | 5-Cl | 2 | $CH_3$ | H | p-Cl | 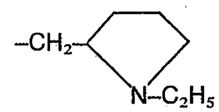 | –<br>H | –<br>Cl | ölig<br>145 |
| 51 | II | H | 4 | H | H | p-Cl | ,, | –<br>H | –<br>Cl | 117<br>126 |
| 52 | II | H | 4 | H | H | p-Cl | 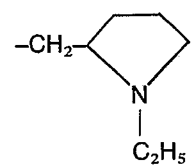 | $CH_3$ | J | 63 |
| 53 | II | H | 2 | H | H | p-Cl | ,, | – | – | 129 |

$A^1$: $OOCC(CH_3)_2–OC_6H_4$-pCl    $A^2$: OOC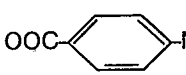$NHCOC(CH_3)_2–OC_6H_4$–pCl

| Beispiel | allgemeine Formel | $R_1$ | Phenoxy-amid-Gruppe in Stellung | $R_3$ | $R_4$ | Z | 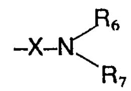 | Quaternisierung bzw. Salzbildung | | Fp °C |
|---|---|---|---|---|---|---|---|---|---|---|
| 54 | II | 2-Cl | 5 | H | H | p-Cl | 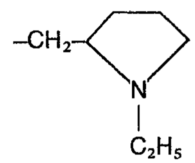 | –<br>H | –<br>Cl | 123<br>110 |
| 55 | II | 5-Cl | 2 | H | H | p-Cl | " | –<br>H | –<br>Cl | 166<br>194 |
| 56 | II | 2-$OCOCH_3$ | 4 | H | H | p-Cl | " | – | – | 168 |
| 57 | II | 2-OH | 4 | H | H | p-Cl | " | – | – | 183 |
| 58 | II | 4-$OCH_3$ | 3 | H | H | p-Cl | " | –<br>H | –<br>Cl | 186<br>196 |
| 59 | II | H | 4 | H | $CH_3$ | p-Cl | " | –<br>H | –<br>Citrat | 137<br>160 |
| 60 | II | 4-Cl | 3 | $CH_3$ | H | p-Cl | " | – | – | ölig |
| 61 | II | 2-Cl | 4 | H | $CH_3$ | p-Cl | " | – | – | ölig |
| 62 | II | H | 2 | H | $CH_3$ | p-Cl | " | – | – | 130 |
| 63 | II | H | 2 | H | $CH_3$ | p-Cl | " | $C_2H_5$ | J | 135 |
| 64 | II | 4-$OCH_3$ | 3 | $CH_3$ | $CH_3$ | p-Cl | " | –<br>H | –<br>Cl | ölig<br>137 |
| 65 | II | 2-Cl | 5 | $CH_3$ | $CH_3$ | p-Cl | " | –<br>H | –<br>Cl | 108<br>200 |
| 66 | II | 2-$OCOCH_3$ | 4 | $CH_3$ | $CH_3$ | p-Cl | " | – | – | ölig |
| 67 | II | 2-OH | 4 | $CH_3$ | $CH_3$ | p-Cl | " | – | – | ölig |

| | | | | | | | | | | |
|---|---|---|---|---|---|---|---|---|---|---|
| 68 | II | 2-OH | 4 | $CH_3$ | $CH_3$ | p-Cl | 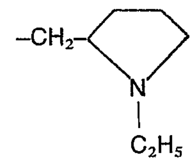 | $CH_3$ | J | 217 |
| 69 | II | H | 4 | $CH_3$ | $CH_3$ | p-Cl | " | –<br>H<br>H | –<br>Cl<br>Citrat | 142<br>58<br>73 |
| 70 | II | H | 3 | $CH_3$ | $CH_3$ | p-Cl | " | –<br>H | –<br>Cl | ölig<br>153 |
| 71 | II | H | 2 | $CH_3$ | $CH_3$ | p-Cl | " | – | – | 104 |
| 72 | II | 5-Cl | 2 | $CH_3$ | $CH_3$ | p-Cl | " | –<br>H | –<br>Cl | 94<br>234 |
| 73 | II | 2-Cl | 4 | H | H | p-Cl | 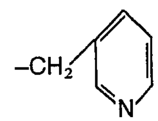 | – | – | 150 |
| 74 | II | 4-Cl | 3 | H | H | p-Cl | " | –<br>H | –<br>Cl | 212<br>222 |
| 75 | II | H | 4 | $CH_3$ | $CH_3$ | p-Cl | " | –<br>H | –<br>Cl | 185<br>143 |
| 76 | II | H | 4 | $CH_3$ | $CH_3$ | p-Cl | 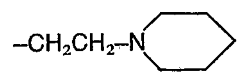 | –<br>H<br>H | –<br>Cl<br>Tartrat | 129<br>193<br>66 |
| 77 | II | 4-$OCH_3$ | 3 | H | H | p-Cl | 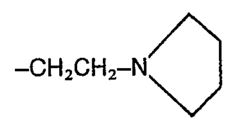 | – | – | 176 |
| 78 | II | H | 4 | $CH_3$ | $CH_3$ | p-Cl | 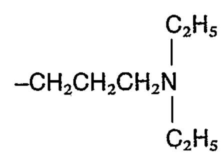 | –<br>H | –<br>Citrat | 144<br>78 |

Die Herstellung der ringgeschlossenen Verbindungen gemäss allgemeiner Formel (III) wird in den folgenden Beispielen beschrieben. Entsprechend den beschriebenen Verfahren hergestellte weitere Verbindungen sind in der anschliessenden Tabelle insgesamt tabellarisch erfasst.

Beispiel 79

5,0 g (0,014 Mol) 2-(p-Chlorphenoxyacetamido)-4-chlor-benzoylchlorid werden in 100 ml Chloroform mit 1,7 g (0,014 Mol) 2-Diäthylaminoäthylamin versetzt und der Ansatz 8 Stunden unter Rückfluss erwärmt. Nach dem Abkühlen wird mit verdünnter Natronlauge sowie Wasser gewaschen und das Lösungsmittel im Vakuum abdestilliert. Der verbleibende Rückstand wird aus Diisopropyläther kristallisiert, wobei 3,3 g (53,7%) 2-(p-Chlorphenoxymethyl)-3-(2-diäthylaminoäthyl)-7-chlor-4(3H)-chinazolinon vom Fp 104 °C erhalten werden.

Elementaranalyse:
$C_{21}H_{23}Cl_2N_3O_2$ (420,4)

| | | | |
|---|---|---|---|
| Ber.: | C 59,99 | H 5,52 | N 9,99 |
| Gef.: | C 59,88 | H 5,32 | N 10,16 |

Hydrochlorid Fp 188 °C.

Beispiel 80

8,1 g (0,02 Mol) der Base nach Beispiel 4 vom Fp 109 °C werden in einem Reaktionsgefäss 15 Minuten trocken auf 180–200 °C erwärmt, wobei gebildetes Wasser durch angelegtes schwaches Vakuum gegen Ende der Reaktionszeit abgesaugt wird. Nach dem Abkühlen wird die Schmelze aus Diisopropyläther kristallisiert, wobei 7,05 g 2-(p-Chlorphenoxymethyl)-3-(2-diäthylaminoäthyl)4-(3H)-chinazolinon vom Fp 85 °C erhalten werden.

Elementaranalyse:
$C_{21}H_{24}ClN_3O_2$ (385,9)

| | | | |
|---|---|---|---|
| Ber.: | C 65,36 | H 6,26 | N 10,89 |
| Gef.: | C 64,72 | H 6,25 | N 10,72 |

Hydrochlorid Fp 194 °C.

| Beispiel | allgemeine Formel | $R_1$ | Phenoxyamid-Gruppe in Stellung | $R_3$ | $R_4$ | Z | $-X-N{<}^{R_6}_{R_7}$ | Quaternisierung bzw. Salzbildung | | Fp °C |
|---|---|---|---|---|---|---|---|---|---|---|
| 79 | III | Cl | | H | H | p-Cl | $-CH_2CH_2N{<}^{C_2H_5}_{C_2H_5}$ | –<br>H | –<br>Cl | 104<br>188 |
| 80 | III | H | | H | H | p-Cl | $-CH_2CH_2N{<}^{C_2H_5}_{C_2H_5}$ | –<br>H | –<br>Cl | 85<br>194 |
| 81 | III | Cl | | H | H | p-Cl | $-CH_2CH_2N{<}^{C_2H_5}_{C_2H_5}$ | $CH_3$ | J | 171 |
| 82 | III | H | | H | H | p-Cl | $-CH_2CH_2N{<}^{C_2H_5}_{C_2H_5}$ | $CH_3$ | J | 100 |
| 83 | III | H | | H | $CH_3$ | p-Cl | $-CH_2CH_2N{<}^{C_2H_5}_{C_2H_5}$ | H | Cl | 180 |
| 84 | III | Cl | | H | H | p-Cl | $-CH_2CH_2N{<}^{C_2H_5}_{C_2H_5}$ | –<br>H | –<br>Citrat | 158<br>141 |
| 85 | III | H | | $CH_3$ | H | p-Cl | $-CH_2CH_2N{<}^{C_2H_5}_{C_2H_5}$ | – | – | ölig |
| 86 | III | H | | H | $CH_3$ | p-Cl | 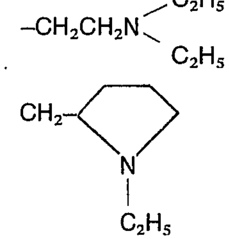 | – | – | ölig |
| 87 | III | H | | H | H | p-Cl | | –<br>H | –<br>Cl | ölig<br>140 |
| 88 | III | Cl | | H | H | p-Cl | | –<br>H | –<br>Cl | 297<br>137 |

**Patentansprüche**

1. Aminobenzoesäurederivate der allgemeinen Formel

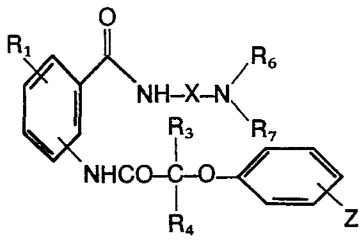

II

oder

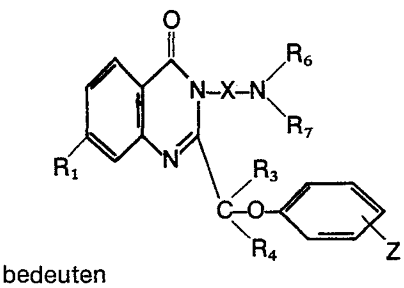

III

worin bedeuten

$R_1$ Wasserstoff, Chlor, Hydroxy, Acetoxy oder $C_1$–$C_3$-Alkoxy;

$R_3$ Wasserstoff oder Methyl,

$R_4$ Wasserstoff, $C_1$–$C_3$-Alkyl oder mit Z substituiertes Phenoxy, mit der Massgabe, dass in Formel (III) mindestens einer der Reste $R_3$ und $R_4$ ein Wasserstoffatom darstellt.

Z Halogen oder Trifluormethyl

X $C_1$–$C_3$-Alkylen

$R_6$ und $R_7$ Äthyl oder zusammen mit dem Stickstoffatom eine

N-Pyrrolidinyl ( 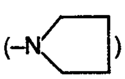 ),

N-Piperidinyl ( 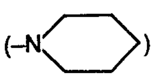 ),

N-Äthyl-2-pyrrolidinyl ( 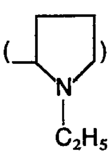 ) oder

3-Pyridinyl ( 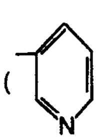 ) -Gruppe

sowie deren mit gegebenenfalls Phenyl-substituierten $C_1$–$C_4$-Alkyl- oder $C_1$–$C_4$-Alkenyl-Gruppen enthaltenden Quaternisierungsmitteln oder pharmazeutisch verträglichen Säuren erhaltenen Derivate.

2. Aminobenzoesäurederivate gemäss Anspruch 1 als pharmazeutische Wirkstoffe.

3. Aminobenzoesäurederivate gemäss Anspruch 1 als antilipämische Wirkstoffe.

4. Verfahren zur Herstellung der Aminobenzoesäurederivate gemäss Anspruch 1, dadurch gekennzeichnet, dass man eine Carbonsäure der allgemeinen Formel

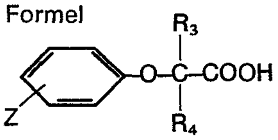

IV

worin Z, $R_3$ und $R_4$ die in Anspruch 1 angegebenen Bedeutungen besitzen oder ein reaktionsfähiges Derivat dieser Carbonsäure mit einer Aminobenzoesäure der allgemeinen Formel

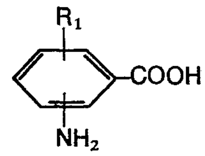

V

in der $R_1$, die in Anspruch 1 angegebene Bedeutung besitzt oder deren reaktionsfähigem Derivat zur Reaktion bringt und die erhaltene Aminobenzoesäure der allgemeinen Formel

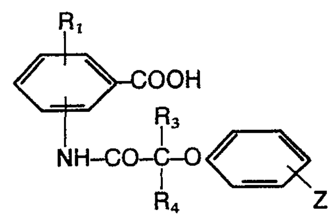

VI

oder deren reaktionsfähiges Derivat mit einem Diamin der allgemeinen Formel

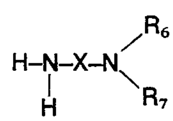

VII

worin X, $R_6$ und $R_7$ die in Anspruch 1 angegebene Bedeutung haben, umsetzt und sodann gegebenenfalls quaternisiert, oder dass man die Aminobenzoesäure der allgemeinen Formel (V) oder deren reaktionsfähiges Derivat, zweckmässig unter Einführung eines Formyl- oder Acetylrestes als Schutzgruppe für den $NH_2$-Rest, zunächst mit dem Diamin der allgemeinen Formel (VII) umsetzt und nach Entfernen der Schutzgruppe mit der Carbonsäure der allgemeinen Formel (IV) oder deren reaktionsfähigem Derivat zur Reaktion bringt.

5. Arzneimittel, dadurch gekennzeichnet, dass es ein Aminobenzoesäurederivat gemäss Anspruch 1 und übliche Träger und/oder Hilfsstoffe enthält.

**Claims**

1. Aminobenzoic acid derivatives having the general formula

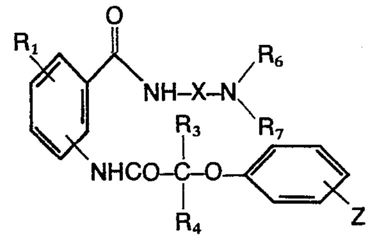

II

or

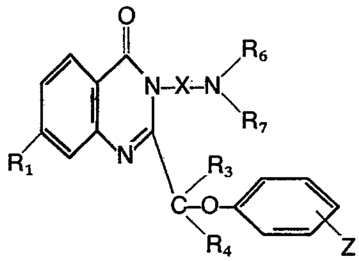

III

in which

$R_1$ means hydrogen, chlorine, hydroxy, acetoxy or $C_1$–$C_3$-alkoxy;

$R_3$ means hydrogen or methyl;

$R_4$ means hydrogen, $C_1$–$C_3$-alkyl or phenoxy substituted with Z, with the proviso, that in formula (III) at least one of the residues $R_3$ an $R_4$ is a hydrogen atom,

Z means halogen or trifluormethyl

X means $C_1$–$C_3$-alkylene

$R_6$ and $R_7$ mean ethyl or together with the nitrogen atom a

N-pyrrolidinyl (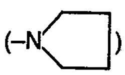),

N-piperidinyl (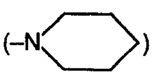),

N-ethyl-2-pyrrolidinyl (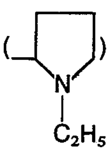) or

3-Pyridinyl (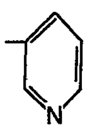) -group

and their derivatives with optionally phenyl-substituted $C_1$–$C_4$-alkyl- or $C_1$–$C_4$-alkenyl-groups containing quarternisation agents or with pharmaceutically acceptable acids.

2. Aminobenzoic acid derivatives according to claim 1 as pharmaceutical agents.

3. Aminobenzoic acid derivatives according to claim 1 as antilipemic agents.

4. Process for the preparation of the aminobenzoic acid derivatives according to claim 1 characterized in that a carboxylic acid of the general formula

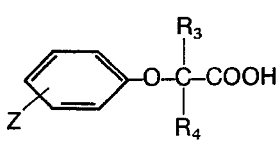

IV

in which Z, $R_3$ and $R_4$ have the meanings given in claim 1 or a reactive derivative of these carboxylic acids are reacted with a aminobenzoic acid of the general formula

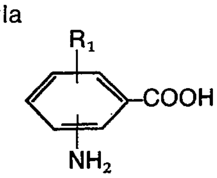

V

in which $R_1$ has the meaning given in claim 1 or with a reactive derivative there of and the aminobenzoic acid obtained having the general formula

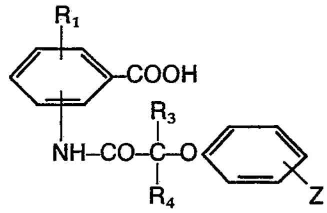

VI

or their reactive derivative is reacted with a diamine of the general formula

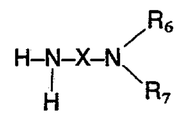

VII

in which X, $R_6$ and $R_7$ have the meaning given in claim 1 followed optionally by quarternisation, or in that the aminobenzoic acid of the general formula (V) or their reactive derivative, suitably while introducing a formyl or acetyl residue as protective group for the $NH_2$-residue, is first reacted with the diamine of the general formula (VII) and after removing of the protective group with the carboxylic acid of the general formula (IV) or their reactive derivative.

5. Pharmaceutical composition characterized in that it contains an aminobenzoic acid derivative according to claim 1 and usual carriers and/or adjuvants.

**Revendications**

1. Dérivés d'acides aminobenzoïques de formule générale

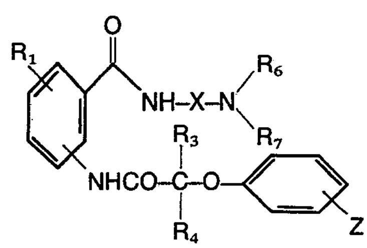

II

ou

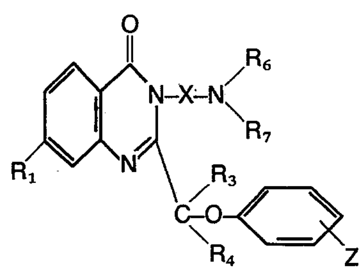

III

dans laquelle les différents symboles ont les significations suivantes:

$R_1$ hydrogène, chlore, hydroxy, acétoxy ou alcoxy à 1–3 C;

$R_3$ hydrogène ou méthyle;

$R_4$ hydrogène, alcoyle à 1–3 C ou phénoxy substitué par Z, avec cette condition que, dans la formule (III), l'un au moins des radicaux $R_3$ et $R_4$ représente un atome d'hydrogène;

Z halogène ou trifluorométhyle;
X alcoylène à 1–3 C;
$R_6$ et $R_7$ éthyle ou, en combinaison avec l'atome d'azote, un groupe

N-pyrrolidinyle (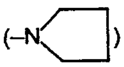),

N-pipéridinyle (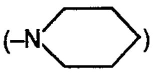),

N-éthyl-2-pyrrolidinyle (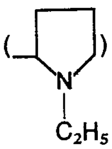) ou

3-pyridinyle (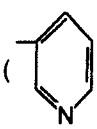),

ainsi que leurs dérivés obtenus avec des agents de quaternisation contenant des groupes alcoyle à 1–4 C ou alcényle à 1–4 C éventuellement substitués par un phényle ou avec des acides acceptables pharmaceutiquement.

2. Dérivés d'acides aminobenzoïques selon la revendication 1, utilisés comme substances actives pharmaceutiques.

3. Dérivés d'acides aminobenzoïques selon la revendication 1, utilisés comme substances actives antilipémiques.

4. Procédé pour la préparation de dérivés d'acides aminobenzoïques selon la revendication 1, caractérisé en ce qu'on met en réaction un acide carboxylique de formule générale

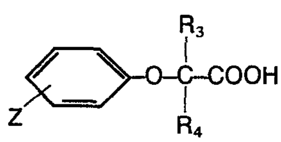 IV

dans laquelle Z, $R_3$ et $R_4$ ont les significations données dans la revendication 1, ou un dérivé réactif de cet acide carboxylique, avec un acide aminobenzoïque de formule générale

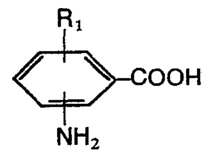 V

dans laquelle $R_1$ a la signification donnée dans la revendication 1, ou un dérivé réactif de cet acide, puis on fait réagir l'acide aminobenzoïque obtenu, de formule générale

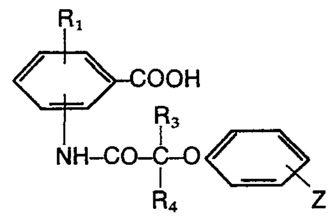 VI

ou un dérivé réactif de celui-ci, avec une diamine de formule générale

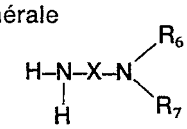 VII

dans laquelle X, $R_6$ et $R_7$ ont les significations données dans la revendication 1, puis on quaternise éventuellement, ou en ce qu'on fait réagir tout d'abord l'acide aminobenzoïque de formule générale (V) ou son dérivé réactif, de préférence avec introduction d'un radical formyle ou acétyle en tant que groupe de protection pour le radical $NH_2$, avec la diamine de formule générale (VII) et, après élimination du groupe de protection, on met en réaction le produit obtenu avec l'acide carboxylique de formule générale (IV) ou son dérivé réactif.

5. Médicament, caractérisé en ce qu'il contient un dérivé d'acide aminobenzoïque selon la revendication 1 et des excipients et/ou adjuvants usuels.